# EUROPÄISCHE PATENTSCHRIFT

(11) **EP 1 506 744 B1**
(45) Veröffentlichungstag und Bekanntmachung des Hinweises auf die Patenterteilung: **10.10.2012**
(21) Anmeldenummer: 04019092.8
(22) Anmeldetag: 11.08.2004
(51) Int. Cl.: A61B 17/22

(54) **Lithotripter**
Lithotripter
Lithotripteur

(30) Priorität: 14.08.2003 DE 10337523
(43) Veröffentlichungstag der Anmeldung: 16.02.2005
(73) Patentinhaber: Dornier MedTech Systems GmbH, 82234 Wessling (DE)
(72) Erfinder: Buchbauer, Peter, 85748 Garching (DE); Grözinger, Reiner, 82239 Alling (DE); Haas, Anton, 81827 München (DE); Hermeking, Hajo, Dr., 88677 Markdorf (DE); Hofsäss, Siegfried, 82140 Olching (DE)
(74) Vertreter: Grünecker, Kinkeldey, Stockmair & Schwanhäusser

(56) Entgegenhaltungen:
- EP-A- 0 739 609
- DE-A- 10 236 177
- DE-A1- 3 737 858
- DE-A1- 19 639 979
- US-A- 3 790 805
- US-A- 5 283 823
- US-A1- 2003 078 523

## Beschreibung

Die vorliegende Erfindung betrifft einen Lithotripter, mit welchem beispielsweise Konkremente im Körper eines Patienten behandelbar sind.

Es sind Lithotripter bekannt, die eine Traganordnung zum Tragen eines Therapiekopfes aufweisen. Die Traganordnung ist mit Hilfe eines Vertikalgelenkes um eine vertikale Achse verstellbar. Ferner ist an einem Horizontalarm, welcher an dem Vertikalgelenk angeordnet ist, ein Horizontalgelenk vorgesehen. Mit dem Horizontalgelenk ist ein den Therapiekopf haltender Bügel um eine horizontale, in Längsrichtung des Horizontalarmes verlaufende Achse schwenkbar. Der Therapiekopf ist an dem Bügel um eine zu der Horizontalachse senkrechte, die Horizontalachse schneidende Achse schwenkbar. Bei dieser Anordnung ist es aufwendig, den Therapiekopf in verschiedene Ausrichtungen bezüglich geneigter Körperflächen eines Patienten zu bringen.

Die DE 299 23 561 U1 betrifft einen Lithotripter mit einer mehrere Armkomponenten aufweisenden Traganordnung, welche über ein Vertikalgelenk in mehrere Arbeitsstellungen schwenkbar an einer Basiseinheit des Lithotripters gelagert ist. Es sind drei Armkomponenten vorgesehen, bei welchen eine erste Armkomponente höhenverstellbar und um eine vertikale Achse schwenkbar an der Basiseinheit vorgesehen ist. Eine zweite Armkomponente ist um eine zweite vertikale Achse schwenkbar an der ersten Armkomponente angelenkt. Eine dritte Armkomponente ist über ein Horizontalgelenk, welches die vertikale Achse zwischen der ersten und der zweiten Armkomponente schneidet, und um eine horizontale Achse schwenkbar ist. Die dritte Armkomponente fungiert als Haltearm für einen Therapiekopf, der wiederum über eine zu der horizontalen Achse senkrecht stehende zusätzliche Achse schwenkbar am Ende des Haltearms angeordnet ist. Durch diese vielen nacheinander geschalteten Arme ist der Therapiekopf in einem großen Raumbereich in viele Stellungen bringbar, so dass ein Arzt den Therapiekopf ohne Verfahren der Basiseinheit oder Verlagern eines Patienten flexibel in viele Behandlungspositionen bringen kann.

Die DE 37 37 858 A1 offenbart einen Lithotripter, dessen Stoßwellenapplikator mit Therapieeinheit über ein Horizontalgelenk an einem Sockel unterhalb eines Arbeitstisches gelagert ist. Ferner ist ein Schräggelenk vorgesehen, mit welchem die Therapieeinheit um eine schräg zu der horizontalen Drehachse verlaufende schräge Drehachse isozentrisch um den Fokus der Therapieeinheit schwenkbar ist. Mit Hilfe des Horizontalgelenkes und des Schräggelenkes ist die Therapieeinheit in zwei Untertischpositionen als Arbeitsstellungen und zwei demgegenüber verschwenkten, auch unterhalb des Tisches befindlichen Parkposition verschwenkbar.

Die nachveröffentlichte DE 102 36 177 A1 betrifft einen Lithotripter, welcher ein Positioniersystem hat, das eine Therapieeinrichtung trägt. Die Therapieeinrichtung ist um ihre eigene Achse drehbar und ferner mit Hilfe einer C-förmigen Verschiebelagerung verschiebbar.

Der vorliegende Erfindung liegt die Aufgabe zugrunde, eine Traganordnung eines Lithotripters so zu verbessern, dass der Therapiekopf auf möglichst einfache Weise mit einer ausgewogenen Kinematik, die einfach bedienbar ist, gut an Körperstellen eines Patienten positionierbar ist.

Die Aufgabe wird erfindungsgemäß gelöst mit einem Lithotripter mit den Merkmalen des Anspruches 1.

Mit Hilfe des Vertikalgelenkes ist ein einfaches Wechseln der Traganordnung zwischen der Arbeitsstellung und der Parkposition zum Transportieren und Lagern des Lithotripters möglich. Das Horizontallager ermöglicht insbesondere ein Schwenken des Therapiekopfes zwischen einer Untertischposition und einer Übertischposition bezüglich einer mit dem Lithotripter verwendeten Patientenliege. Gleichzeitig ist es in der Arbeitsstellung der Traganordnung einfach, den Therapiekopf durch Schwenken um die schräg im Raum verlaufende Achse aus verschiedenen schrägen Raumrichtungen, in die gewünschte Position zu Körperflächen eines Patienten zu bringen. Dadurch, dass sich die horizontale Achse, die Wirkungsachse des Therapiekopfes und die Schrägachse schneiden, ist der Therapiekopf beim Drehen um die Schrägachse isozentrisch zu diesem Schnittpunkt bewegbar und bleibt auf diesen Schnittpunkt ausgerichtet. Die horizontale Achse, die Schrägachse und die Wirkungsachse schneiden sich etwa im Bereich eines Fokus des Therapiekopfes. Damit ist der Therapiekopf sowohl um die Schrägachse als auch um die horizontale Achse isozentrisch um den Fokus bewegbar.

Bevorzugterweise kann die Schrägachse stets windschief zu der vertikalen Achse verlaufen.

Günstigerweise kann die vertikale Achse von der Basiseinheit horizontal beabstandet angeordnet sein. Dies ermöglicht ein Schwenken des Querarmes über eine parallele Lage zu der Basiseinheit hinaus nahe an die Basiseinheit heran.

Besonders vorteilhaft kann der Therapiekopf in eine Position relativ oberhalb oder auf der Basiseinheit bringbar sein. Hiermit ist eine bezüglich eines seitlichen Bereiches der Basiseinheit besonders raumsparende Parkposition möglich, wobei in einer Vertikalprojektion der Basiseinheit die Traganordnung und/oder der Therapiekopf nur bereichsweise über die Basiseinheit übersteht.

Bevorzugt kann an der Basiseinheit eine separate Stütze vorgesehen sein, mit welcher der Therapiekopf und/oder die Halteanordnung abstützbar sind. Mit der Stütze ist die Halteanordnung von ihrem eigenen Gewicht und dem Gewicht des Therapiekopfes entlastbar, was einem Verstellen oder Verformen der Traganordnung entgegenwirkt, z. B. in der Parkposition.

Besonders vorteilhaft kann das Schräggelenk in bestimmten Winkelintervallen verrastbar sein. Dies ermöglicht ein schnelles Auffinden von und Arretieren in häufig genutzten Stellungen des Schräggelenkes.

Besonders günstig können die Winkelintervalle Winkel von etwa 5° bis 20° umfassen, vorzugsweise etwa 10°. Damit sind besonders oft auftretende Verstellintervalle abgedeckt.

Vorteilhafterweise kann das Horizontalgelenk in bestimmten Winkelintervallen verrastbar sein. Hierdurch ist ein schnelles Auffinden und Arretieren häufiger Stellungen des Horizontalgelenkes möglich.

Bevorzugt können die Winkelintervalle Winkel von etwa 5° bis 20° umfassen, vorzugsweise etwa 10°. Mit diesen Winkelintervallen sind in der Praxis besonders oft genutzte Positionen des Horizontalgelenkes schnell einstellbar.

Vorzugsweise kann das Vertikalgelenk in bestimmten Winkelstellungen verrastbar sein. Damit ist ein schnelles Finden und Arretieren von häufigen Stellungen des Vertikalgelenkes möglich.

Günstigerweise können die Winkelstellungen wenigstens eine Parkposition und/oder eine Arbeitsstellung umfassen. Hierdurch ist ein schneller Wechsel der Traganordnung zwischen Parkpositionen und der Arbeitsposition möglich.

In einer weiteren Ausführungsform der Erfindung können der Querarm und das Horizontalgelenk wenigstens eine bündige Seitenabschlussfläche miteinander ausbilden. Diese Seitenabschlussfläche kann als bündige Anlagefläche beim Heranschwenken des Querarmes an die Basiseinheit des Lithotripters dienen.

Bevorzugterweise können an dem Haltearm zwei Laserpointer vorgesehen sein. Die von den Laserpointern ausgesandten Laserstrahlen sind zum Bestimmen der Lage des Haltearmes im Raum nutzbar. Dies kann beispielsweise zum Bestimmen der Lage des Haltearmes relativ zu einem C-Bogen einer Röntgenvorrichtung dienen, wenn der Lithotripter mit einer solchen Röntgenvorrichtung kombiniert wird.

Günstigerweise kann ein erster Laserpointer an einem Horizontalgelenkteil des Haltearmes an einer der Abzweigungsstelle des Haltearmes von dem Horizontalgelenk etwa gegenüberliegenden Seite des Horizontalgelenkteiles vorgesehen sein. Bei einem Bewegen des Haltearmes verändert der vom ersten Laserpointer ausgesandten Strahl entsprechend seine Lage, beispielsweise bei einem Schwenken von einer Über- in eine Untertischposition. Wird der Lithotripter in Kombination mit einer Röntgenvorrichtung mit C-Bogen verwendet, kann dies zum Bestimmen und eventuellen Korrigieren der Lage des Haltearmes relativ zu dem C-Bogen genutzt werden.

Besonders bevorzugt kann ein zweiter Laserpointer an einem therapiekopfsseitigen Teil des Haltearmes an einer dem Therapiekopf etwa gegenüberliegenden Seite vorgesehen sein. Bei einem Bewegen des Haltearmes verändert sich die Lage des von dem zweiten Laserpointer ausgesandten Laserstrahles entsprechend, z. B. bei einem Schwenken von einer Über- in eine Untertischposition. In Kombination des Lithotripters mit einer Röntgenvorrichtung mit C-Bogen ist die beispielsweise zum Analysieren und eventuellen Korrigieren der Lage des Haltearmes relativ zu dem C-Bogen nutzbar.

Besonders günstig kann der Strahl des zweiten Laserpointers entlang der Schrägachse verlaufen. Hiermit ist die Lage der Schrägachse im Raum bestimmbar.

Bevorzugt können die Laserstrahlen der beiden Laserpointer etwa in einer Ebene des Haltearmes verlaufen. Dies ermöglicht ein Bestimmen der Lage der Ebene des Haltearmes im Raum, bei Verwenden einer Röntgenvorrichtung mit C-Bogen beispielsweise auch zum Projizieren der Lagerstrahlen auf den C-Bogen.

Besonders bevorzugt können die Laserstrahlen der beiden Laserpointer etwa in entgegengesetzte Richtungen weisen. Damit ist ein besonders präzises Bestimmen der Lage des Haltearmes im Raum möglich, z. B. gegenüber einem C-Bogen einer Röntgenvorrichtung, welche zusammen mit dem Lithotripter verwendet werden kann.

Eine Ausführungsform der vorliegenden Erfindung ist in der Zeichnung dargestellt. Es zeigen:
- Figur 1: eine perspektivische Ansicht auf die vordere, die rechte und die obere Seite des erfindungsgemäßen Lithotripters in einer Arbeitsstellung,
- Figur 2 und 3: perspektivische Ansichten auf die vordere, die rechte und die obere Seite des erfindungsgemäßen Lithotripters in einer Parkposition,
- Figur 4: eine perspektivische Ansicht auf die vordere und die obere Seite des erfindungsgemäßen Lithotripters in einer Parkposition,
- Figur 5: eine perspektivische Ansicht auf die vordere, die rechte und die obere Seite einer Geräteanordnung mit dem erfindungsgemäßen Lithotripter und einer Röntgenvorrichtung sowie einer Patientenliege,
- Figur 6: eine Ansicht auf die rechte Seite der Geräteanordnung aus Figur 5, und
- Figur 7: eine Ansicht auf die vordere Seite der Geräteanordnung aus Figur 5.

Im Folgenden beziehen sich Bezeichnungen wie "horizontal", "vertikal" oder "Bodenbereich" auf einen Boden 80, auf dem ein erfindungsgemäßer Lithotripter und gegebenenfalls eine Röntgenvorrichtung und eine Patientenliege stehen.

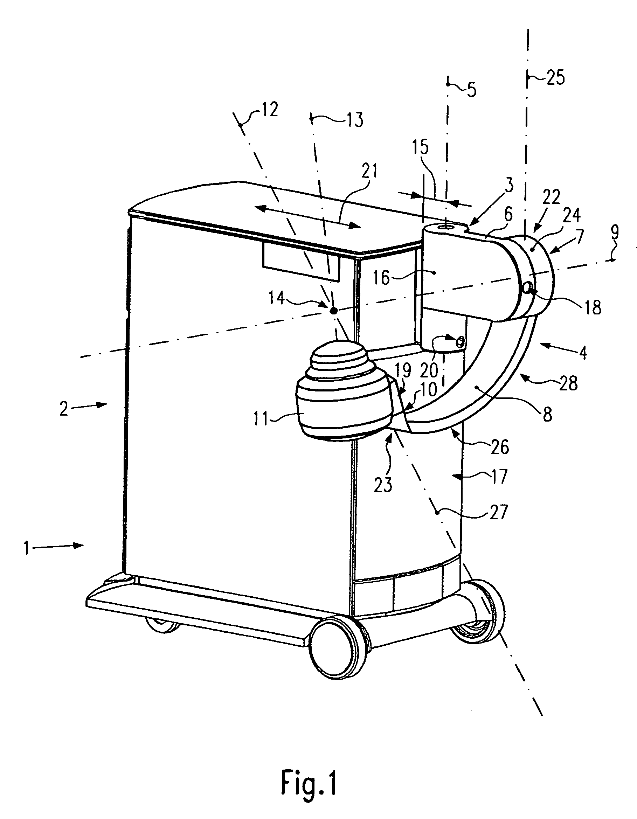

Figur 1 zeigt eine perspektivische Ansicht auf die vordere, die rechte und die obere Seite eines erfindungsgemäßen Lithotripters 1. Der Lithotripter 1 weist eine Basiseinheit 2 auf, an welcher über ein Vertikalgelenk 3 eine Traganordnung 4 um eine etwa vertikale Achse 5 schwenkbar angelenkt ist. Die Traganordnung 4 weist einen an dem Vertikalgelenk 3 angeordneten Querarm 6 auf, an welchem ein Horizontalgelenk 7 vorgesehen ist. Über das Horizontalgelenk 7 ist ein etwa C-bogenförmiger Haltearm 8 um eine etwa horizontale Achse 9 schwenkbar an dem Querarm 6 angelenkt. Die horizontale Achse 9 verläuft etwa senkrecht zu der vertikalen Achse 5.

Über ein Schräggelenk 10 ist an dem Haltearm 8 ein Therapiekopf 11 um eine schräg im Raum verlaufende Achse 12 schwenkbar angelenkt. Mit dem Therapiekopf 11 sind Stoßwellen erzeugbar, welche durch Lenken eines auf einer Hauptwirkungslinie 13 liegenden Fokus 14 gebündelt werden. Die schräg im Raum verlaufende Achse 12, die horizontale Achse 9 und die Hauptwirkungslinie 13 schneiden einander, wobei ihr Schnittpunkt mit dem Fokus 14 zusammenfällt. Die schräg im Raum verlaufende Achse 12 ist stets windschief zu der vertikalen Achse 5.

Die vertikale Achse 5 verläuft um einen Abstand 15 beabstandet zu der Basiseinheit 2. Damit kann der mit dem Horizontalgelenk 7 eine bündige Seitenabschlussfläche 16 ausbildende Querarm 6 über eine etwa parallele Lage hinaus an eine rechte Vorderseite 17 der Basiseinheit 2 heranbewegt werden. Hierbei kann die bündige Seitenabschlussfläche 16 des Querarmes 6 an der Basiseinheit 2 anliegen.

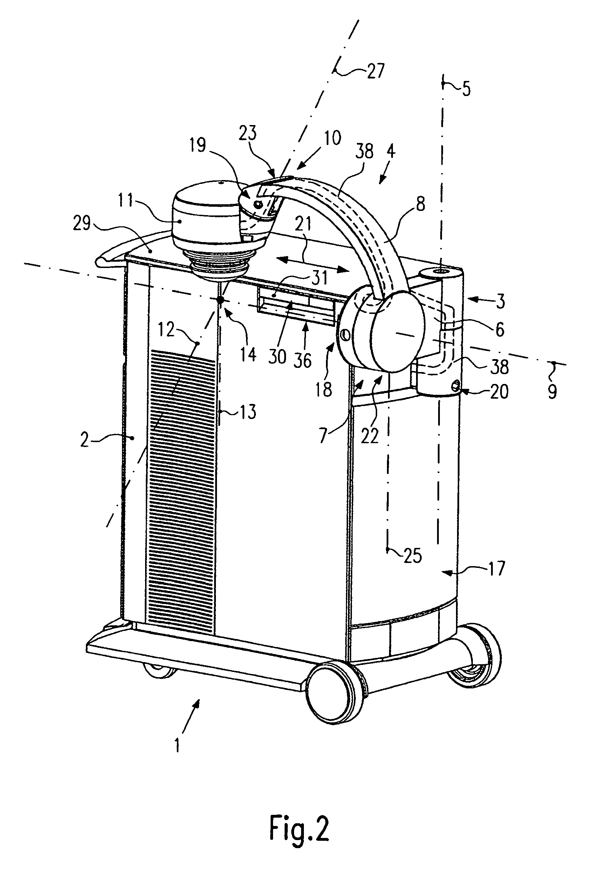

Das Vertikalgelenk 3, der Querarm 6, das Horizontalgelenk 7, der Haltearm 8 und das Schräggelenk 10 sind großvolumig ausgeführt, wobei durch ihr Inneres ein Versorgungsstrang 38 von der Basiseinheit 2 zu dem Therapiekopf 11 geführt ist. Der Versorgungsstrang 38 ist in Figur 2 in gestrichelten Linien angedeutet. Der Versorgungsstrang 38 weist eine Wasserführung und eine Stromzufuhr auf. Über die Stromzufuhr wird auch eine Stoßwellenquelle des Therapiekopfes 11 mit Hochspannung versorgt. Das Vertikalgelenk 3 und die Traganordnung 4 sind dabei so ausgebildet, dass der Versorgungsstrang 38 in ihrem Inneren mit genügend Spiel und über ausreichend große Radien geführt ist, insbesondere im Hinblick auf die im Versorgungsstrang 38 enthaltenen Kabel. Durch das innenseitige Führen des Versorgungsstranges 38 können die Traganordnung 4 und der Therapiekopf 11 behinderungsfrei bedient werden. Gleichzeitig ist der Versorgungsstrang 38 vor Beschädigungen geschützt.

In dem Vertikalgelenk 3, dem Horizontalgelenk 7 und dem Schräggelenk 10 können Vierpunkt-Kugellager zum Einsatz kommen.

Figur 1 zeigt die Einheit aus Therapiekopf 11 und Haltearm 8 in einer Untertischposition. Dabei befinden sich der Therapiekopf 11 und der Haltearm 8 im Wesentlichen unterhalb der horizontalen Achse 9. Mit Hilfe des Horizontaldrehgelenkes 7 kann die Einheit aus Therapiekopf 11 und Haltearm 8 in eine Übertischposition gebracht werden, in der sich der Therapiekopf 11 und der Haltearm 8 im Wesentlichen oberhalb der horizontalen Achse 9 befinden.

Befindet sich der Haltearm 8, wie gezeigt, in einer tiefstmöglichen Position gegenüber der horizontalen Achse 9, befindet sich der Haltearm 8 in einer untersten Untertischposition. Befindet sich der Haltearm in einer höchstmöglichen Lage gegenüber der horizontalen Achse 9, nimmt er eine oberste Übertischposition ein.

Mit Hilfe einer Verrasteinrichtung des Horizontalgelenkes 7, von der in Figur 1 ein Horizontalverrastknopf 18 gezeigt ist, ist der Haltearm 8 in verschiedenen Schwenkpositionen um die horizontale Achse 9 verrastbar. Dabei können Rastintervalle mit Winkel von etwa 5° bis 20° vorgesehen sein, vorzugsweise ein Winkel von etwa 10°. Beispielsweise können die oberste Über- und die unterste Untertischposition als Raststellung vorgesehen sein. Der Haltearm 8 kann zusätzlich in Stellungen verrastbar sein, in denen er gegenüber der obersten Über- und untersten Untertischposition um jeweils ± 10° um die horizontale Achse 9 verschwenkt ist.

Auch an dem Schräggelenk 10 ist eine Verrasteinrichtung vorgesehen, von welcher in Figur 1 nur ein Schrägverrastknopf 19 gezeigt ist. Mit der Schrägverrasteinrichtung ist der Therapiekopf 11 gegenüber dem Haltearm 8 in verschiedenen Schwenkpositionen um die schräg im Raum verlaufender Achse 12 verrastbar. Das Verrasten kann in Winkelintervallen von etwa 5° bis 20° erfolgen, vorzugsweise in Winkelintervallen von etwa 10°.

Ebenso weist das Vertikalgelenk 3 eine Vertikalverrasteinrichtung auf. Mit Hilfe der Vertikalverrasteinrichtung, von der in Figur 1 nur ein Vertikalverrastknopf 20 gezeigt ist, ist der Querarm 6 in verschiedenen Schwenkpositionen um die vertikale Achse 5 verrastbar. Bevorzugt ist der Querarm 6 in einer Stellung verrastbar, in welcher seine Längsrichtung parallel zu einer Längsrichtung 21 der Basiseinheit 2 des Lithotripters 1 ist. In dieser Raststellung befindet sich die Traganordnung 4 in ihrer Arbeitsstellung. Dabei verläuft die horizontale Achse 9 etwa senkrecht zu der Längsrichtung 21 der Basiseinheit 2.

Ferner ist der Querarm 6 in einer an die Basiseinheit 2 herangeschwenkten Stellung verrastbar, in welcher die horizontale Achse 9 etwa parallel zu der Längsrichtung 21 der Basiseinheit 2 verläuft. In dieser Raststellung befindet sich die Traganordnung 4 in einer ersten Parkposition, wenn der Therapiekopf 11 und der Haltearm 8 etwa in oberster Übertischposition angeordnet ist.

Gegenüber der genannten an die Basiseinheit 2 herangeschwenkten Lage des Querarmes 6 kann der Querarm 6 zusätzlich oder alternativ in einer noch weiter an die Basiseinheit 2 herangeschwenkten Stellung verrastbar sein. Befinden sich dabei der Therapiekopf 11 und der Haltearm 8 etwa in ihren obersten Übertischpositionen, befindet sich die Traganordnung 4 in jeweils einer zweiten Parkposition.

Die Verrasteinrichtungen sind jeweils so aufgebaut, dass eine verrastete Stellung durch drücken des jeweiligen Verrastknopfes lösbar ist. Bei einem Bewegen des jeweiligen Elementes um die betreffende Achse erfolgt ein Verrasten mit nicht gedrücktem Verrastknopf durch automatisches Einrasten einer Rastsperre.

Es können zusätzlich oder alternativ fixe oder einstellbare Rastintervalle vorgesehen sein, mit welchen die jeweiligen Elemente in Stellungen verrastbar sind, die beispielsweise von einem Anwender häufig genutzt werden. Hierdurch ist ein schnelles Wechseln zwischen und arretieren in den von dem Anwender häufig genutzten Stellungen möglich. Das entsprechende Bedienen des Lithotripters 1 ist somit einfach und schnell möglich.

An dem Haltearm 8 sind zwei Laserpointer 22, 23 vorgesehen. Der erste Laserpointer 22 ist an einem zu dem Haltearm 8 gehörenden Horizontaldrehgelenkteil 24 an einer Position etwa gegenüberliegend zu der Abzweigung des Haltearmes 4 an diesem Horizontaldrehgelenkteil vorgesehen. Ein von dem ersten Laserpointer 22 erzeugter Laserstrahl 25 verläuft in Arbeitsstellung des Haltearmes 8 in etwa vertikaler Richtung und somit etwa parallel zu der vertikalen Achse 5.

Der zweite Laserpointer 23 ist an einem therapiekopfseitigen Teil 26 des Haltearmes 8 an einer dem Therapiekopf 11 etwa gegenüberliegenden Seite vorgesehen. Ein von dem zweiten Laserpointer 23 erzeugter zweiter Laserstrahl 27 verläuft entlang der schräg im Raum verlaufenden Achse 12. Dementsprechend sind die beiden Laserstrahlen 25, 27 in jeweils etwa entgegengesetzte Richtungen gerichtet. Die beiden Laserstrahlen 25, 27 verlaufen in einer Ebene 28 des Haltearmes 8, in welcher auch die horizontale Achse 9 und die Hauptwirkungslinie 13 des Therapiekopfes 11 verlaufen.

In Figur 2 ist der Lithotripter 1 in seiner ersten Parkposition gezeigt. In der ersten Parkposition, in der die horizontale Achse 9 etwa parallel zu der Längsrichtung 21 der Basiseinheit 2 verläuft. Hierbei befindet sich der Therapiekopf 11 in einer Position vertikal im Wesentlichen oberhalb der Basiseinheit 2, das heißt im Wesentlichen oberhalb einer Oberseite der Basiseinheit 2. In einer Vertikalprojektion des Profils der Basiseinheit 2 steht dementsprechend nur ein Teil der Einheit aus Therapiekopf 11 und Traganordnung 4 seitlich über die Basiseinheit 2 über.

Da in der ersten Parkposition der Querarm 6 noch nicht an der Basiseinheit 2 anliegt, kann die Einheit aus Therapiekopf 11 und Traganordnung 4 noch weiter in Richtung zu der Basiseinheit 2 geschwenkt werden, wobei der Therapiekopf 11 auf der Oberseite 29 der Basiseinheit 2 zur Auflage kommt. In dieser Position befindet sich die Einheit aus Therapiekopf 11 und Traganordnung 4 in ihrer zweiten Parkstellung.

In der zweiten Parkstellung ist durch das Aufliegen des Therapiekopfes 11 auf der Oberseite der Basiseinheit 2 die Traganordnung 4 wenigstens teilweise von dem Gewicht des Therapiekopfes 11 und anteilig von ihrem eigenen Gewicht entlastet.

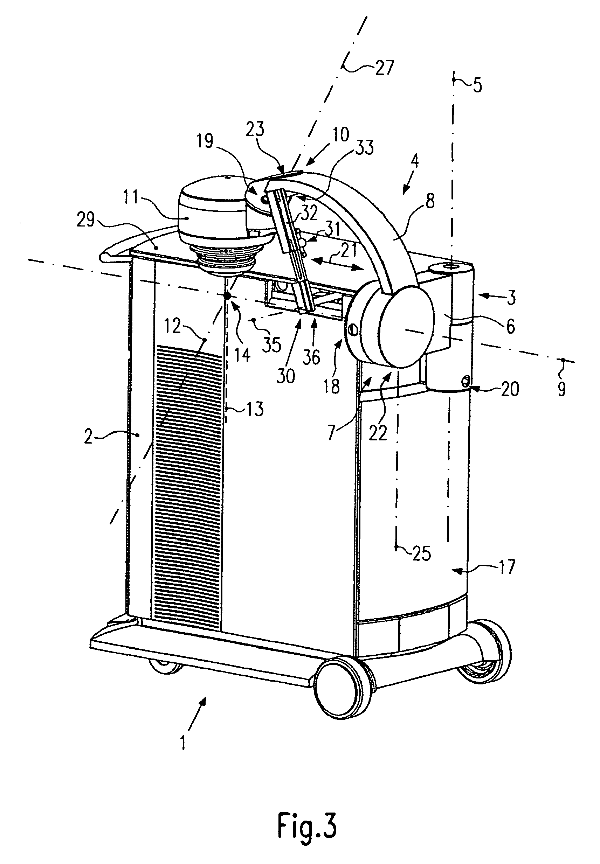

An der Basiseinheit 2 ist eine separate, auszieh- und hochklappbare Stütze 30 vorgesehen. Ein Greifteil 31 der Stütze 30 ist in seiner Längsrichtung teleskopierbar. In Figur 3 ist die Stütze 30 in aus- und hochgeschwenkten Zustand gezeigt, wobei ein Stützabschnitt 32 des teleskopierten Greifteiles 31 mit einem Querelement 33 den Tragarm 8 im Bereich des Schräggelenkes 10 an einer Innenseite 34 abstützt. Die Einheit aus Therapiekopf 11 und Traganordnung 4 befindet sich in der ersten Parkstellung.

Durch die Schwenkbarkeit der Stütze 30 um eine etwa horizontale Achse 35, ist der Stützabschnitt 32 und/oder das Querelement 33 der Stütze 30 an verschiedenen Positionen des Haltearmes 8 und/oder des Schräggelenkes 10 und/oder den Therapiekopf 11 stützend in Anlage bringbar.

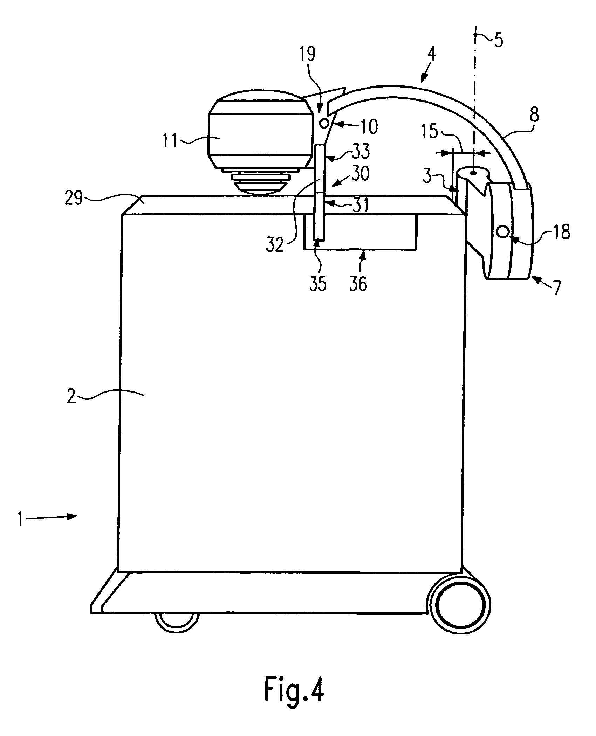

Die Lage der horizontalen Achse 35 der Stütze 30 kann variierbar sein. Gemäß der Darstellung von Figur 3 befindet sich die horizontale Achse 35 durch eine Stützenöffnung 36 verlaufend bezüglich der Stützenöffnung 36 näher an dem Vertikalgelenk 3. In der Darstellung gemäß Figur 4 befindet sich die horizontale Achse 35 bezüglich der Stützenöffnung 36 in einer weiter von dem Vertikalgelenk beabstandeten Position. Das Greifteil 31 befindet sich hier in einem nicht teleskopierten Zustand, wobei das Querelement 33 einen Teil des Schräggelenkes 10 stützt. Auch hierbei befindet sich die Einheit aus Therapiekopf 11 und Traganordnung 4 in ihrer ersten Parkposition.

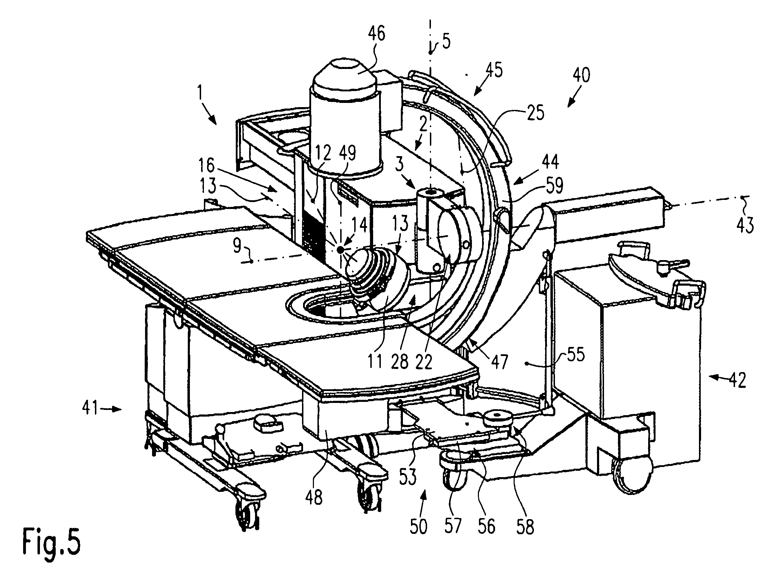

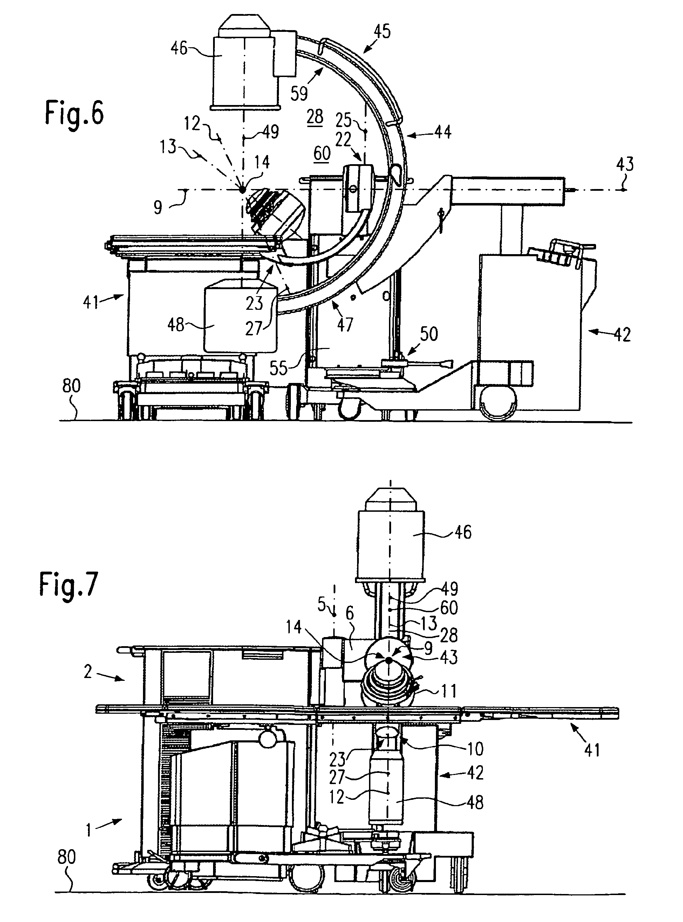

In den Figuren 5 bis 7 ist eine modular koppelbare Geräteanordnung 40 mit dem erfindungsgemäßen Lithotripter 1, einer Röntgenvorrichtung 42 und einer Patientenliege 41 gezeigt. Die Röntgenvorrichtung 42 weist einen um eine etwa horizontale Achse 43 schwenkbar gelagerten C-Bogen 44 auf, der an einer ersten Hälfte 45 eine Röntgenquelle 46 trägt und an einer zweiten Hälfte 47 einen Bildverstärker 48. Von der Röntgenquelle 46 zu dem Bildverstärker 48 verläuft beim Betrieb der Röntgenvorrichtung 42 ein Hauptröntgenstrahl 49, welcher einen zentralen Teil des Wirkungsbereiches der Röntgenvorrichtung 42 repräsentiert. Der Hauptröntgenstrahl schneidet die etwa horizontale Achse 43 des C-Bogens 44.

In den Darstellungen der Figuren 5 bis 7 befindet sich der Lithotripter in der in Figur 1 gezeigten Arbeitsstellung. Ferner befindet sich die Röntgenvorrichtung 42 in einer Arbeitsstellung, wobei ihr Hauptröntgenstrahl 49 in etwa vertikaler Richtung verläuft. Mit Hilfe einer Kopplungsanordnung 50 sind der Lithotripter 1 und die Röntgenvorrichtung 42 in einer Relativlage zueinander positioniert und arretierend gekoppelt, in welcher der Schnittpunkt des Hauptröntgenstrahls 49 der Röntgenvorrichtung 42 mit der horizontalen Achse 43 der Röntgenvorrichtung 42 mit dem Isozentrum 14 des Lithotripters 1 zusammenfällt. Dementsprechend befinden sich der Lithotripter 1 und die Röntgenvorrichtung 42 in ihren Arbeitsstellungen in einer isozentrischen Ausrichtung zueinander.

Die Kopplungsanordnung 50 besteht im Wesentlichen aus einer ersten Kopplungskomponente 51, die zu dem Lithotripter 1 gehört, und einer zweiten Kopplungskomponente 52, die zu der Röntgenvorrichtung 42 gehört. Die erste Kopplungskomponente 51 weist eine Halteplatte 53 auf, die um eine etwa horizontale, zu der Längsrichtung der Basiseinheit 2 etwa quer verlaufende Achse 54 zwischen einer Transportstellung und einer Kopplungsstellung zum Koppeln mit der zweiten Kopplungskomponente 52 schwenkbar ist. In ihrer Transportstellung kann die erste Kopplungskomponente 51 von einer Fronttür 55 der Basiseinheit 2 verdeckt werden, welche in der Darstellung von Figur 5 geöffnet gezeigt ist.

Die zweite Kopplungskomponente 52 weist einen wenigstens abschnittsweise keilartig ausgebildeten Schuh auf, welcher in einer an der Halteplatte 53 vorgesehenen keilförmigen Ausnehmung eines Ausnehmungskörpers selbstzentrierend in Anlage bringbar ist. Die erste und die zweite Kopplungskomponente 51, 52 sind mit Hilfe eines an der Oberseite vorstehenden Haltevorsprungs und einer an der zweiten Kopplungskomponente vorgesehenen Exzenterspannvorrichtung in gekoppeltem Zustand arretierend verspannbar. Zum Verspannen wird der Haltevorsprung durch eine Führungsausnehmung der Exzenterspannvorrichtung geführt.

Bei der in den Figuren 5 bis 7 gezeigten isozentrischen Anordnung des Lithotripters 1 und der Röntgenvorrichtung 42 zueinander, befindet sich der C-Bogen 44 in einer Position, in der sein Hauptröntgenstrahl 49 etwa vertikal verläuft. Der Haltearm 8 befindet sich in seiner untersten Untertischposition. Dementsprechend fallen die Ebene 28 des Haltearmes 8 und eine durch den Hauptröntgenstrahl 49 und die horizontale Achse 43 des C-Bogens 44 definierte Ebene 60 des C-Bogens 44 zusammen. Folglich trifft der erste Laserstrahl 25 im Bereich der ersten Hälfte 45 des C-Bogens 44 auf eine Innenseite 59 des C-Bogens 44. Der zweite Laserstrahl trifft entsprechend im Bereich der zweiten Hälfte 47 des C-Bogens 44 auf die Innenseite 59 des C-Bogens 44.

Befindet sich der Haltearm 8 in seiner obersten Übertischposition, trifft der erste Laserstrahl 25 im Bereich der zweiten Hälfte 47 auf die Innenseite 59 und der zweite Laserstrahl 27 im Bereich der ersten Hälfte 45 auf die Innenseite 59 des C-Bogens 44.

## Patentansprüche

1. Lithotripter (1) mit einer einen Therapiekopf (11) tragenden Traganordnung (4), welche über ein Vertikalgelenk (3) um eine etwa vertikale Achse (5) zwischen einer Arbeitsstellung und wenigstens einer Parkposition schwenkbar an einer Basiseinheit (2) des Lithotripters (1) gelagert ist und einen Haltearm (8) aufweist, welcher über ein Horizontalgelenk (7) um eine etwa quer zu einem an dem Vertikalgelenk (3) gelagerten Querarm (6) der Traganordnung (4) verlaufende etwa horizontale Achse (9) schwenkbar ist, wobei an dem Haltearm (8) ein Schräggelenk (10) der Traganordnung (4) vorgesehen ist, mit welchem der Therapiekopf (11) um eine zu der horizontalen Achse (9) und zu der vertikalen Achse (5) schräg angeordnete und durch das Schräggelenk (10) verlaufende Schrägachse (12) schwenkbar ist, und der Therapiekopf (11) eine Wirkungsachse (13) und einen Fokus (14) hat, um welchen der Therapiekopf (11) mit Hilfe des Horizontalgelenkes (7) isozentrisch zwischen einer Untertischposition, in welcher sich der Therapiekopf (11) und der Haltearm (8) im Wesentlichen unterhalb der horizontalen Achse (9) befinden, und einer Übertischposition, in welcher sich der Therapiekopf (11) und der Haltearm (8) im Wesentlichen oberhalb der horizontalen Achse (9) befinden, schwenkbar ist, wobei sich die horizontale Achse (9), die Schrägachse (12) und die Wirkungsachse (13) etwa im Bereich des Fokus (14) des Therapiekopfes (11) schneiden und die vertikale Achse (5) mittels des Querarmes (6) stets zur horizontalen Achse (9) beabstandet ist.

2. Lithotripter nach einem der Ansprüche 1,
**dadurch gekennzeichnet,**
**dass** die Schrägachse (12) stets windschief zu der vertikalen Achse (5) verläuft.

3. Lithotripter nach wenigstens einem der vorhergehenden Ansprüche,
**dadurch gekennzeichnet,**
**dass** die vertikale Achse (5) von der Basiseinheit (2) horizontal beabstandet (15) angeordnet ist.

4. Lithotripter nach wenigstens einem der vorhergehenden Ansprüche,
**dadurch gekennzeichnet,**
**dass** der Therapiekopf (11) in einer Position vertikal oberhalb oder auf der Basiseinheit (2) bringbar ist.

5. Lithotripter nach wenigstens einem der vorhergehenden Ansprüche,
**dadurch gekennzeichnet,**
**dass** an der Basiseinheit (2) eine separate Stütze (30) vorgesehen ist, mit welcher der Therapiekopf (11) und/oder die Halteanordnung (4) abstützbar ist.

6. Lithotripter nach Anspruch 5,
**dadurch gekennzeichnet,**
**dass** die Stütze (30) teleskopierbar ist.

7. Lithotripter nach wenigstens einem der vorhergehenden Ansprüche,
**dadurch gekennzeichnet,**
**dass** das Schräggelenk (10) in bestimmten Winkelintervallen verrastbar ist.

8. Lithotripter nach Anspruch 7,
**dadurch gekennzeichnet,**
**dass** die Winkelintervalle Winkel von etwa 5° bis 20° umfassen, vorzugsweise etwa 10°.

9. Lithotripter nach wenigstens einem der vorhergehenden Ansprüche,
**dadurch gekennzeichnet,**
**dass** das Horizontalgelenk (7) in bestimmten Winkelintervallen verrastbar ist.

10. Lithotripter nach Anspruch 9,
**dadurch gekennzeichnet,**
**dass** die Winkelintervalle Winkel von etwa 5° bis 20° umfassen, vorzugsweise etwa 10°.

11. Lithotripter nach wenigstens einem der vorhergehenden Ansprüche,
**dadurch gekennzeichnet,**
**dass** das Vertikalgelenk (3) in bestimmten Winkelstellungen verrastbar ist.

12. Lithotripter nach Anspruch 11,
**dadurch gekennzeichnet,**
**dass** die Winkelstellungen wenigstens eine Parkposition und/oder eine Arbeitsstellung umfassen.

13. Lithotripter nach wenigstens einem der vorhergehenden Ansprüche,
**dadurch gekennzeichnet,**
**dass** der Querarm (6) und das Horizontalgelenk (7) wenigstens eine bündige Seitenabschlussfläche (16) miteinander ausbilden.

## Claims

1. Lithotripter (1) with a support arrangement (4) supporting a treatment head (11) which is mounted on a base unit (2) of the lithotripter (1) so that it can be pivoted by means of a vertical link (3) about an approximately vertical axis (5) between an operating position and at least one stowed position and which has a retaining arm (8) which can be pivoted by means of a horizontal link (7) about an approximately horizontal axis (9) extending transversely to a transverse arm (6) of the support arrangement (4) mounted on the vertical link (3), and an angular contact link (10) of the support arrangement (4) is provided on the retaining arm (8) by means of which the treatment head (11) can be pivoted about an angled axis (12) disposed at an angle with respect to the horizontal axis (9) and to the vertical axis (5) and extending through the angular contact link (10), and the treatment head (11) has an action axis (13) and a focal point (14) about which the treatment head (11) can be isocentrally pivoted with the aid of the horizontal link (7) between a below-table position in which the treatment head (11) and retaining arm (8) are disposed essentially underneath the horizontal axis (9) and an above-table position in which the treatment head (11) and retaining arm (8) are disposed essentially above the horizontal axis (9), and the horizontal axis (9), angled axis (12) and action axis (13) intersect approximately in the region of the focal point (14) of the treatment head (11) and the vertical axis (5) is always spaced at a distance from the horizontal axis (9) by means of the transverse arm (6).

2. Lithotripter as claimed in claim 1,
**characterised in that**
the angled axis (12) always extends in a skewed position relative to the vertical axis (5).

3. Lithotripter as claimed in at least one of the preceding claims,
**characterised in that**
the vertical axis (5) is disposed horizontally spaced (15) apart from the base unit (2).

4. Lithotripter as claimed in at least one of the preceding claims,
**characterised in that**
the treatment head (11) can be moved into a position vertically above or on the base unit (2).

5. Lithotripter as claimed in at least one of the preceding claims,
**characterised in that**
a separate support (30) is provided on the base unit (2) by means of which the treatment head (11) and/or holding arrangement (4) can be supported.

6. Lithotripter as claimed in claim 5,
**characterised in that**
the support (30) is telescopic.

7. Lithotripter as claimed in at least one of the preceding claims,
**characterised in that**
the angular contact link (10) can be latched in specific angular intervals.

8. Lithotripter as claimed in claim 7,
**characterised in that**
the angular intervals comprise angles of approximately 5° to 20°, preferably approximately 10°.

9. Lithotripter as claimed in at least one of the preceding claims,
**characterised in that**
the horizontal link (7) can be latched in specific angular intervals.

10. Lithotripter as claimed in claim 9,
**characterised in that**
the angular intervals comprise angles of approximately 5° to 20°, preferably approximately 10°.

11. Lithotripter as claimed in at least one of the preceding claims,
**characterised in that**
the vertical link (3) can be latched in specific angular positions.

12. Lithotripter as claimed in claim 11,
**characterised in that**
the angular positions comprise at least one stowed position and/or a working position.

13. Lithotripter as claimed in at least one of the preceding claims,
**characterised in that**
the transverse arm (6) and the horizontal link (7) form at least one flush side terminating surface (16) with one another.

## Revendications

1. Lithotripteur (1) avec un dispositif porteur (4) qui porte une tête de traitement (11), qui est monté par l'intermédiaire d'une articulation verticale (3) sur une unité de base (2) du lithotripteur (1) pour pouvoir pivoter, sur un axe (5) à peu près vertical, entre une position de travail et au moins une position de repos, et qui présente un bras de support (8) apte à pivoter par l'intermédiaire d'une articulation horizontale (7) sur un axe (9) à peu près horizontal qui s'étend transversalement par rapport à un bras transversal (6), monté sur l'articulation verticale (3), du dispositif porteur (4), étant précisé qu'il est prévu sur le bras de support (8) une articulation inclinée (10) du dispositif porteur (4), avec laquelle la tête de traitement (11) est apte à pivoter sur un axe incliné (12) qui est incliné par rapport à l'axe horizontal (9) et à l'axe vertical (5) et qui traverse ladite articulation inclinée (10), et que la tête de traitement (11) a un axe d'action (13) et un point focal (14) sur lequel ladite tête de traitement (11) est apte à pivoter de manière isocentrique, à l'aide de l'articulation horizontale (7), entre une position sous table dans laquelle la tête (11) et le bras de support (8) se trouvent globalement au-dessous de l'axe horizontal (9), et une position sur table dans laquelle la tête (11) et le bras (8) se trouvent globalement au-dessus de l'axe horizontal (9), étant précisé que l'axe horizontal (9), l'axe incliné (12) et l'axe d'action (13) se coupent à peu près dans la zone du point focal (14) de la tête de traitement (11), et que l'axe vertical (5) est toujours espacé de l'axe horizontal (9) grâce au bras transversal (6).

2. Lithotripteur selon la revendication 1, **caractérisé en ce que** l'axe incliné (12) s'étend toujours en biais par rapport à l'axe vertical (5).

3. Lithotripteur selon l'une des revendications précédentes, **caractérisé en ce que** l'axe vertical (5) est espacé à l'horizontale (15) de l'unité de base (2).

4. Lithotripteur selon l'une des revendications précédentes, **caractérisé en ce que** la tête de traitement (11) est apte à être amenée dans une position où elle se trouve à la verticale au-dessus de l'unité de base (2) ou sur celle-ci.

5. Lithotripteur selon l'une des revendications précédentes, **caractérisé en ce qu'**il est prévu sur l'unité de base (2) un montant séparé (30) avec lequel la tête de traitement (11) et/ou le dispositif de support (4) peuvent être supportés.

6. Lithotripteur selon la revendication 5, **caractérisé en ce que** le montant (30) est télescopique.

7. Lithotripteur selon l'une des revendications précédentes, **caractérisé en ce que** l'articulation inclinée (10) est apte à être arrêtée par encliquetage suivant des intervalles angulaires définis.

8. Lithotripteur selon la revendication 7, **caractérisé en ce que** les intervalles angulaires comprennent des angles d'environ 5° à 20°, de préférence d'environ 10°.

9. Lithotripteur selon l'une des revendications précédentes, **caractérisé en ce que** l'articulation horizontale (7) est apte à être arrêtée par encliquetage suivant des intervalles angulaires définis.

10. Lithotripteur selon la revendication 9, **caractérisé en ce que** les intervalles angulaires comprennent des angles d'environ 5° à 20°, de préférence d'environ 10°.

11. Lithotripteur selon l'une des revendications précédentes, **caractérisé en ce que** l'articulation verticale (3) est apte à être arrêtée par encliquetage dans des positions angulaires définies.

12. Lithotripteur selon la revendication 11, **caractérisé en ce que** les positions angulaires comprennent au moins une position de repos et/ou une position de travail.

13. Lithotripteur selon l'une des revendications précédentes, **caractérisé en ce que** le bras transversal (6) et l'articulation horizontale (7) forment ensemble au moins une surface terminale latérale continue (16).
